(19) Europäisches Patentamt
European Patent Office
Office européen des brevets

(11) **EP 1 241 464 B1**

(12) **EUROPEAN PATENT SPECIFICATION**

(45) Date of publication and mention
of the grant of the patent:
**29.08.2007 Bulletin 2007/35**

(51) Int Cl.:
***G01N 21/47*** *(2006.01)*  ***G01N 21/64*** *(2006.01)*

(21) Application number: **02251861.7**

(22) Date of filing: **15.03.2002**

(54) **Non-contact optical monitor**

Kontaktloser optischer Monitor

Moniteur optique sans contact

(84) Designated Contracting States:
**AT BE CH CY DE DK ES FI FR GB GR IE IT LI LU MC NL PT SE TR**

(30) Priority: **17.03.2001 GB 0106676**

(43) Date of publication of application:
**18.09.2002 Bulletin 2002/38**

(73) Proprietor: **WRC PLC**
**Swindon,**
**Wiltshire SN5 8YF (GB)**

(72) Inventors:
 • **Schmalfuss, Jürgen Harald**
 **82538 Geretsried (DE)**
 • **Maccraith, Brian Dominic**
 **Portmarnock,**
 **County Dublin (IE)**
 • **O'Connor, Mark Joseph**
 **Castlenock,**
 **Dublin 15 (IE)**
 • **Russell, Stephen Lindsay**
 **Swindon,**
 **Wiltshire SN5 7AP (GB)**

(74) Representative: **Curtis, Philip Anthony et al**
**A.A. Thornton & Co.,**
**235 High Holborn**
**London WC1V 7LE (GB)**

(56) References cited:
GB-A- 2 130 742     US-A- 3 309 956
US-A- 4 668 860     US-A- 5 400 137
US-A- 5 461 236     US-A- 5 489 977

**Description**

This invention relates to the measurement of the characteristics of a liquid sample by an optical non-contact method.

Industrial processes frequently require monitoring of one or more key parameters which characterise the quality of the process material, for example the turbidity or colour of a beverage. Many of these measurements are made rapidly and continuously by online process analysers rather than waiting for the results of sampling the process material and making the measurements off line in a laboratory. Some process materials are particularly difficult to handle such as sewage, dairy products or molten metals and this has given rise to non-contact measurement techniques which seek to make the measurement whilst avoiding direct contact between the measuring system and the process material. There may also be health and safety issues which make non-contact measurement desirable.

An example of such a process is the collection, treatment and discharge of wastewaters. Municipal sewage treatment plants and industrial wastewater treatment plants are required to reduce the polluting load of the waste to an agreed level prior to discharge, and will benefit from continuous measurement of some key pollution parameters such as suspended solids concentration, biochemical oxygen demand (BOD), chemical oxygen demand (COD) and ammonia concentration. Such data is of use both at the discharge of the plant and also at the inlet and within the collection network, where for example significant changes in the polluting load can notify operators of the need to adjust the aeration in the biological stage of the plant. This perceived need has been met by a range of monitoring instruments, some of which are dipped into a flowing channel, others pump a sample through a chemical analyser. The reliability of these instruments is badly affected by the direct contact with the wastewater sample which frequently blocks pipes or coats optical surfaces with layers of grease or biological films or dirt. Further these instruments are frequently complex machines so that the cost of monitoring the wastewater is high both in initial capital cost and the maintenance costs of keeping the instruments operating within acceptable accuracy. Many of these machines require supplies of services such as compressed air or mains water to assist in cleaning, thus incurring both installation and operating costs and restricting their application.

In the prior art a number of non-contact methods have been devised to minimise or eliminate the direct contact between the sample and the measuring equipment. Examples of these are the surface scatter turbidimeter described in US patent number 3,309,956, which uses a special flowcell where the sample forms a mirror-like surface and the sample turbidity is measured by interrogating the surface using a beam of light and a detector which is arranged to measure non-specularly reflected light. A development of this system is described in US patent number 5,400,137 which employs a very similar flowcell arrangement but includes, in addition to the turbidity measurement, a light source and detector for fluorescence measurement for oils or other fluorescent material. Variants on the non-contact principle are the falling column flowcells produced by Sigrist in which the sample is arranged as a continuous falling stream which is interrogated optically to determine turbidity, absorbance or fluorescence. The Sigrist instruments employ light sources and detectors arranged around the falling column, whilst the arrangement described in UK patent number 2,310,282 uses a beam of light which is directed into a falling column from within a header tank above, and then can detect light which is scattered or absorbed by the falling liquid sample. All of these non-contact systems use a sampling system which is itself subject to fouling and blockage. Further, the sampled liquid may not be representative of the main flow, especially where there is a range of different suspended material present.

In US4668860 there is disclosed a scatterometer for evaluating the surface quality of an optical element made from a material that has bulk scatterring of light by measuring the light scattering from the surface.

We have now found a way to overcome these difficulties. Broadly, the invention relates to a device for monitoring one or more characteristics of a liquid sample, as defined in claim 1.

In an embodiment, the device includes at least two sources of collimated radiation arranged side by side, or at least two sources of collimated radiation arranged on a common optical axis.

In an embodiment, the device includes at least two radiation detectors arranged side by side, or at least two radiation detectors arranged on a common optical axis.

Preferably, the each collimated radiation source is a laser.

In an embodiment the device is capable of being operated in a hand-held fashion. To this end the device may be provided with means to enable it to be held by hand, such as a hand grip, and is of a size and weight such that it is readily portable. In this embodiment, it is preferably that the device further comprising means to measure the range from the device to the sample surface, such as a position sensitive detector. It is also desirable that the device further comprises an inclinometer. In this embodiment, the detector is preferably movable relative to the radiation source; an adjustable connection may be provided between the source and the detector, and preferably this connection is a telescopable connection.

Preferably, the device further comprising means to accommodate changes in level of the sample.

Desirably, the radiation source is pivotable and controllable such that the interaction between radiation and sample occurs on or close to the optical axis of the radiation detector.

It is preferred that the angle between the axis of the collimated radiation and the detector optical axis is controlled by the device using a mirror on a motorised rotating shaft such that the interaction between radiation and sample occurs on or close to the optical axis of the radiation detector

In a preferred embodiment, the detector consists of a linear array of detectors, or a single position sensitive detector such that as the level varies the image of the region of interaction of the source radiation and sample moves laterally across the detector.

In a preferred embodiment, the radiation source and radiation detector are mounted together on a float which is free to move on one or more swinging arms or tethers maintaining a constant range between the radiation source and detectors and the sample surface.

Preferably, the device further comprising means for measuring the losses across the optical windows. The means for measuring the losses across the optical windows may include a detector window arranged such that collimated radiation from the radiation source can be passed through the radiation source window and detector optical window directly, without interaction with the sample.

Preferably, the device further comprises a data smoothing means, such as a median filter, for smoothing the data received by the detector. Some applications generate spurious high values for the scatter and fluorescence data caused by for example large ripples, foam and highly reflecting floating objects. The data can be smoothed to remove these spurious data values using the median filter.

In a preferred construction, the device comprises a radiation source housing containing the or each radiation source, a detector housing containing the or each detector and a lens arranged such that radiation from the or each radiation source must pass through the lens in order to reach the or each detector, a support for supporting the detector housing and the radiation source housing, and control means for controlling the or each radiation source and the or each detector.

According to another aspect of the invention there is provided the use of a device, as described above, to measure the polluting properties of an aqueous sample. Thus, the device can be used to infer the suspended solids content of the aqueous sample from measurements of scattered radiation at one or more wavelengths.

The measurement of fluorescence using excitation in the range 360 - 410 nanometres and emission in the range 570 - 630 nanometres can be used to infer the organic load of an aqueous sample which may be expressed as biochemical oxygen demand or chemical oxygen demand.

The measurement of fluorescence using excitation in the range 360 - 410 nanometres and emission in the range 570 - 630 nanometres can be used in combination with measurements of scattered radiation to infer the organic load of an aqueous sample which may be expressed as biochemical oxygen demand or chemical oxygen demand.

The flow velocity of the aqueous sample can be used in combination with other measurements to infer the suspended solids and organic load of the flowing sample.

The measurement of fluorescence, especially at the wavelength ranges indicated above are particularly useful for monitoring sewage, which has been found to respond that these wavelengths. Thus, the invention also provides a method for monitoring sewage using the device described above by measuring fluorescence using excitation and emission in the wavelengths described above.

Reference is now made to the accompanying drawings, in which:

Figures 1 to 8 are schematic side elevations of embodiments of an optical monitor according to the invention;
Figure 9 is a schematic side elevation of a handheld embodiment of an optical monitor according to the invention; and
Figure 10 shows the embodiment of Figure 9 in greater detail.

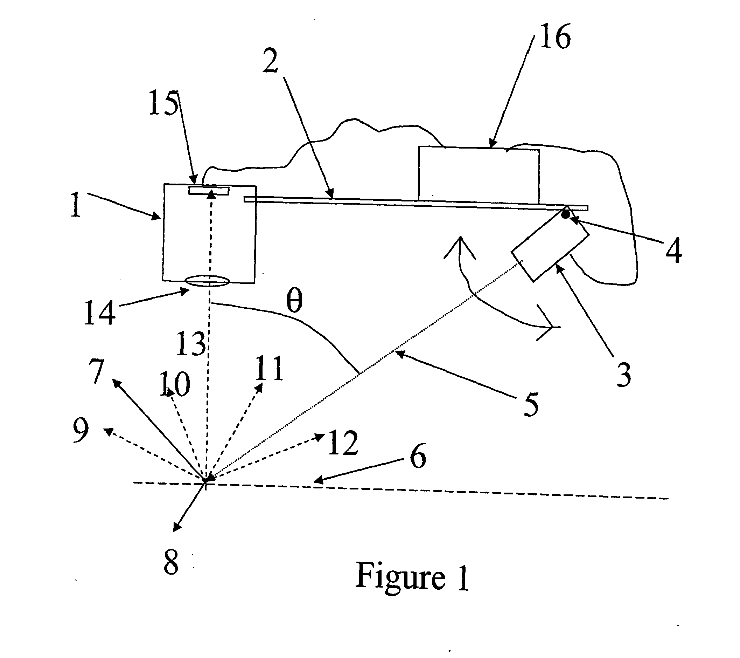

Referring to Figure 1, there is provided an optical detector unit 1 mounted on a support in the form of a rigid bar 2. A collimated radiation source unit 3 is also mounted on the bar 2, and is pivoted about point 4. A beam of radiation 5, from the source 3, strikes the sample surface 6 producing a specular reflected beam 7, and a refracted beam 8. Other processes of interaction of the incident radiation 5 and the sample such as scattering or fluorescence may generate beams of radiation such as 9, 10, 11 and 12; some of this radiation, such as that designated 13 is collected by a lens 14 to be focused on a detector 15 housed within the unit 1.

The unit 3 includes a precision motor or other means of rotating it about the pivot point 4; this rotation is under the control of a computer 16. In an alternative embodiment the movement of the beam of radiation may be achieved by deflecting the radiation beam using a mirror mounted on a rotating shaft. Within the unit 3 there may be more than one radiation source operating at different optical wavelengths and the selection of radiation source is under control of the computer 16. The detector 15 is also connected to the computer 16.

The arrangement is such that the radiation source unit 3 swings around the pivot 4 under control of the computer 16 until the detector 15 registers a maximum response. By this means the interaction of the radiation and the sample may be measured over a range of sample levels, and the range between the detector unit 1 and sample surface 6 can be calculated from the position of the radiation source unit 3 when the maximum signal is registered at the detector 15.

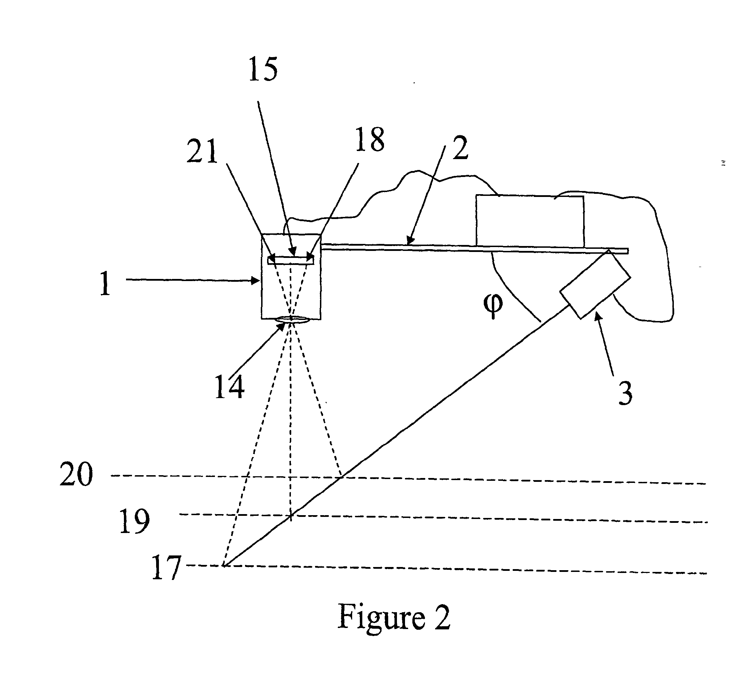

In an alternative embodiment the variations in level can be accommodated without moving parts. Referring to figure 2, the radiation source unit 3 is attached to the rigid member 2 at a fixed angle φ. With the liquid sample at a low position 17, the radiation produced by the interaction with the sample is imaged by lens 14 of detector unit 1 on to the detector 15 at position 18. As the sample level rises to position 19 the radiation produced by the interaction with the sample is imaged by lens 14 centrally on to the detector 15. At a higher sample level 20 the radiation produced by the interaction with the sample is imaged by lens 14 on to the detector at position 21. It may be seen therefore that as the sample level moves vertically, the position of the imaged radiation on the detector moves laterally. If the detector 15 is a position sensitive detector, or an array of detectors, the sample level can be inferred by the established method of triangulation from the position of the radiation falling on the detector 15. At the same time, information about the strength of the interaction between the radiation and sample is available from the intensity of the radiation falling on the detector 15. If an array of detectors is used the progressive decay and spread of the radiation as it penetrates the sample may be measured to gain additional information about the sample.

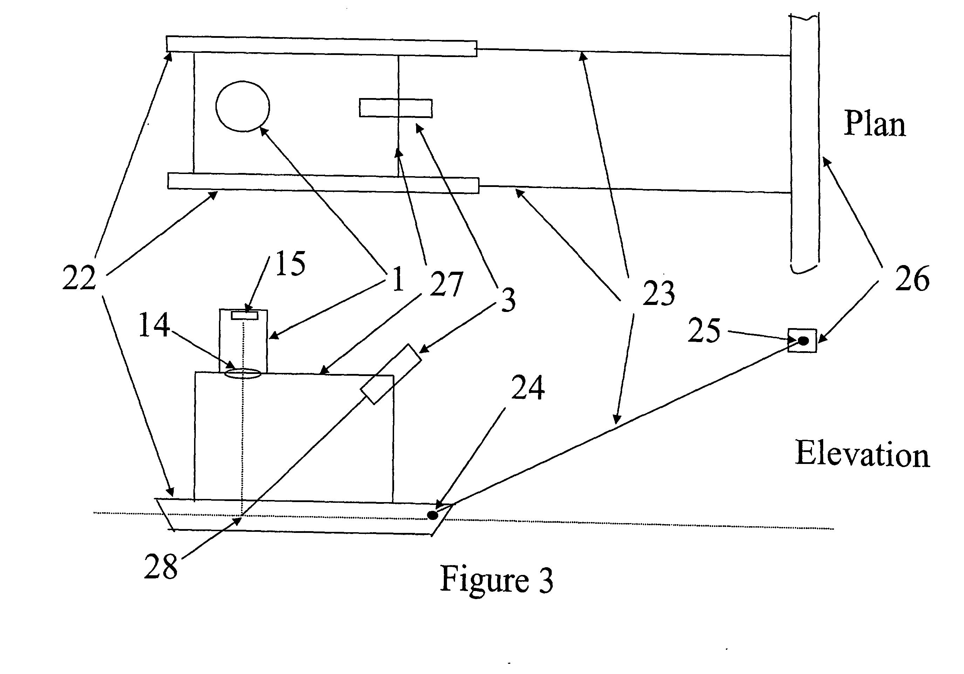

In a further alternative embodiment variations in liquid level are accommodated by allowing the optical system to float on the sample surface, which can only be liquid in this embodiment, maintaining a constant range between the optical system and the sample surface. Referring to figure 3, the optical system is mounted on one or more floats 22 which are attached by one or more arms 23 pivoted at points 24 and 25, or a simple tether to a rigid support 26. The measurement equipment on the floats consists of a radiation source unit 3, which may contain one or more different sources of collimated radiation, and a detector unit 1 mounted on a rigid frame 27 on the float(s) 22. The radiation from the source unit 3 strikes the liquid surface at 28 and interacts with it to produce scattered, fluorescent or other radiation which is radiated upwards from the liquid surface. Some of this radiation is collected by lens 14 which focuses the radiation on a detector 15. As the liquid level changes, the float-mounted equipment rides with the liquid maintaining a constant range between the optical units 3 and 1 and the liquid. The system may include a rotational transducer or other means to monitor the angle between the arms 23 and the float(s) 22 and hence deduce the liquid level. The radiation source unit 3 operates under control of a computer, not shown in this diagram, connected to both the radiation source unit 3 and the detector unit 1. The computer may be mounted on the float or may be mounted with the rigid support on the sides of the channel, tank or other structure.

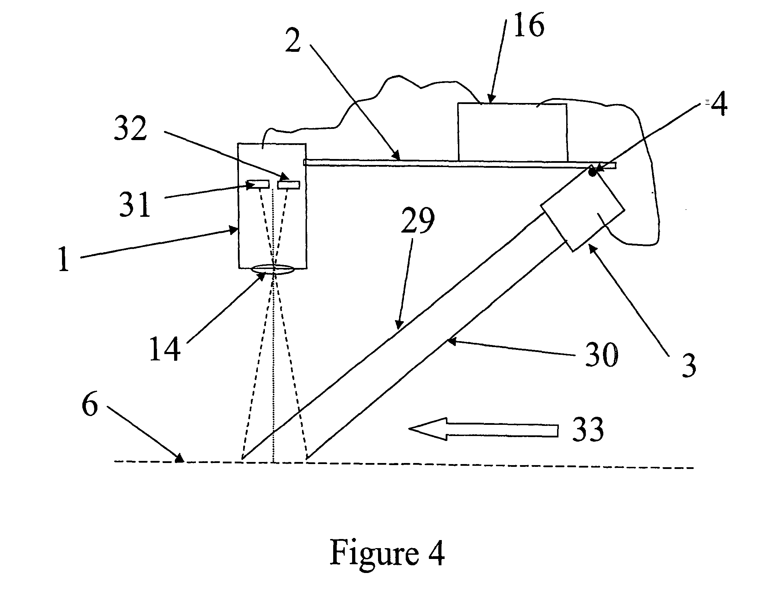

A number of arrangements of radiation sources and detectors are possible within the basic geometries of figures 1, 2 and 3, depending upon what measurements are required to characterise the liquid sample. Referring to figure 4, which uses the configuration of figure 1 by way of example, the radiation source unit 3 generates two or more separate collimated beams of radiation side by side 29 and 30. These strike the sample surface 6 and interact with the sample producing radiation above the sample surface. This radiation is focused by lens 14 and produces images of the regions of interaction on two or more detectors 31 and 32 which are arranged to respond to the radiation of interest for example by the use of suitable filters. Two separate lenses, one for each detector, could be used in place of the single lens 14. In addition to collecting data which characterises the sample 6, this arrangement can be used to measure the surface velocity of the sample along the direction 33 using cross correlation of the signals measured at detectors 31 and 32.

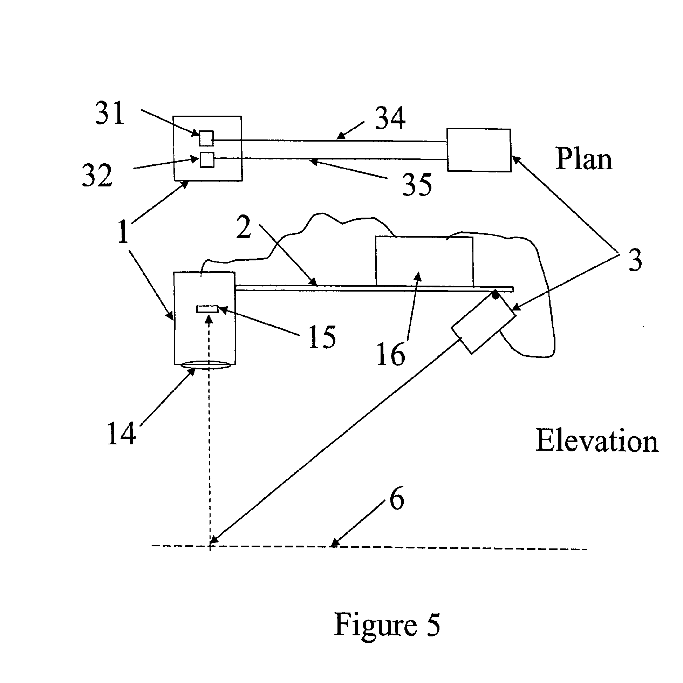

The radiation sources and detectors may be arranged side by side in a plane at right angles to that shown in figure 4. Referring to figure 5, using as an example the configuration of figure 1, two or more collimated beams of radiation 34 and 35 are arranged to emerge side by side from the radiation source unit 3 and strike the sample surface 6 below the detector unit 1. This contains two or more radiation detectors 31 and 32 arranged side by side and with a radiation response suited to the properties of the liquid sample being monitored. Radiation emerging from the sample surface 6 is focused on the detectors 31 and 32 by one or more lenses 14.

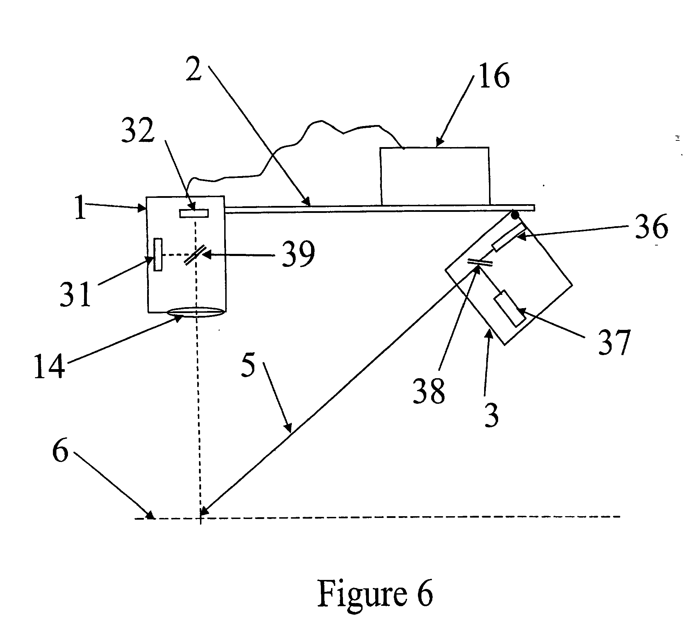

There is a further different possible arrangement of radiation sources and detectors which may be used within the basic geometries of figures 1, 2 and 3, Using the configuration of figure 1 as an example, and referring to figure 6, collimated radiation from 2 separate sources 36 and 37 is arranged to emerge from the radiation source unit 3 along a common axis 5 by the use of a plate 38 which may be part silvered or coated with a wavelength selective layer designed to reflect radiation from source 37 and transmit radiation from source 36 so that the emerging radiation is coaxial. Some of the radiation produced by the interaction with the sample 6 is collected by the lens 14 in the detector unit 1 where it is split by beamsplitter 39 into 2 portions to be registered by detectors 31 and 32. A further variant on this arrangement would be to use a single radiation source and measure the scattered radiation using 1 detector and the fluorescent radiation with the second detector.

It should be appreciated that the different arrangements of figures 1 to 6 may be used to assemble combinations of collimated sources and detectors to suit the measurement requirement.

The collimated radiation sources may be for example lasers. If the collimated radiation source is plane polarised, it is advantageous that the E vector is aligned vertically to the sample surface to obtain maximum transmission at the interface.

It is highly desirable in making the measurements needed to characterise the sample that they do not interfere with each other and are not disturbed by changes in ambient radiation. There are a number of methods by which this can be achieved. One method is to modulate the radiation sources at different frequencies and to use synchronous detection methods such as lock-in amplification to select only the radiation from the sample which was stimulated by interaction with the intended radiation source. It may be advantageous to operate the different radiation sources one at a time to assist in separating the effects which are to be measured.

Changes in the sample level will affect the intensity of the radiation received at the detector unit and it is desirable to correct for this using a computer or other means. Corrections are desirable to compensate for three effects. Referring to figure 1, the first is that the proportions of the incident source radiation to be reflected and refracted at the sample surface will vary with the angle $\theta$ at which the radiation strikes the sample surface and it is only the refracted radiation which will interact with the sample to generate for example scattered or fluorescent radiation. The intensity of radiation received at the detector will fall with increasing $\theta$ according to the known laws of reflection and refraction. The second effect is that the strength of scattered radiation will generally depend upon the angle between the incident radiation and the scatter direction, and this will depend upon the relative size of the particles and the wavelength of the radiation according to the established theories of radiation scattering. The third effect is the inverse square law governing the range between a point radiation source and detector of fixed aperture, which is approximated by this system. Whilst most of these effects can be calculated, a convenient method of correction is to use a lookup table derived from empirical measurements in the computer for example, to normalise the measured radiation intensities for the effects of varying range to the sample.

A further effect which can be compensated for occurs when the depth of sample is sufficiently small that reflected radiation from the floor of the vessel, pipe or channel interferes with the measurement. For example, with crude sewage samples this effect becomes significant with depths of less than about 80 millimetres of sewage depth and causes an increase in the scattered radiation detected. As with the range effect, a lookup table in the computer derived empirically offers a convenient method of applying a correction.

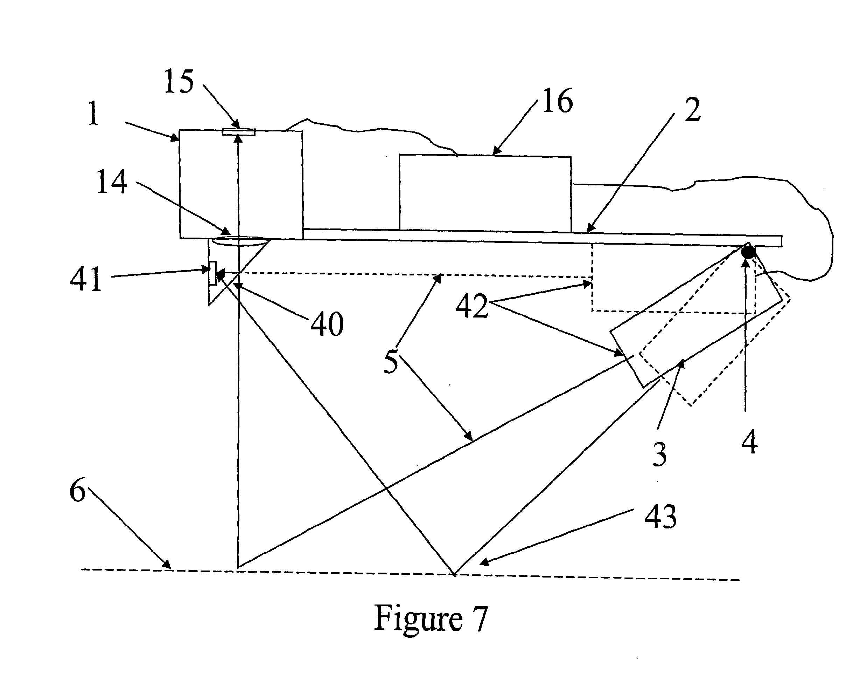

The embodiments described all employ optical windows through which radiation is transmitted and received and these windows may be subject to fouling from dust, condensation, spray, cobwebs and other sources. The optical arrangement of the present invention may be used to provide a measure of the degree of window fouling and thus to apply a correction for radiation losses at the windows. Referring to figure 7, the radiation source unit 3 is shown in the normal measuring position using solid lines, where the collimated radiation beam 5 strikes the sample surface 6 and radiation from the interaction between sample and radiation enters the main detector 15 through the inclined window 40. The radiation source unit 3 may be raised under control of the computer 16 to the position shown by the dotted lines so that the collimated beam 5 enters the window 40 directly and reaches the auxiliary detector 41. The measurements of intensity by the main detector 15 will vary with: the strength of the collimated source beam inside the radiation source unit 3; the attenuation by the radiation source unit window 42; the attenuation due to the radiation path in air due to mist or other losses; attenuation due to the detector unit window 40. The measurement made by the auxiliary detector with the radiation source unit in the raised position will therefore allow a correction to be applied which improves the accuracy of the measurement in the presence of some optical window fouling. It has the added benefit of providing registration for the angular position measurement of the radiation source unit relative to the detector unit. Additional information may be gathered by moving the radiation source to the position below the normal operating position also shown in broken lines, such that the collimated radiation strikes the sample at 43 and is reflected specularly from the sample surface into the auxiliary detector 41. The radiation intensity at the main detector 15 will depend upon the source intensity; losses at the windows 42 and 40; losses in the transmission path; losses upon reflection. Provided that the reflection characteristics of the sample 6 are known, the reflection losses may be calculated from the angle of incidence $\alpha$ which is known from the rotational position of the source unit 1. The measurements made in the two dotted positions may then be compared and provide a means of measuring and correcting for transmission losses in the air path. This lower position for the radiation source unit 3 also provides an alternative method for measuring the sample level to the usual method with the radiation source in the normal measuring position.

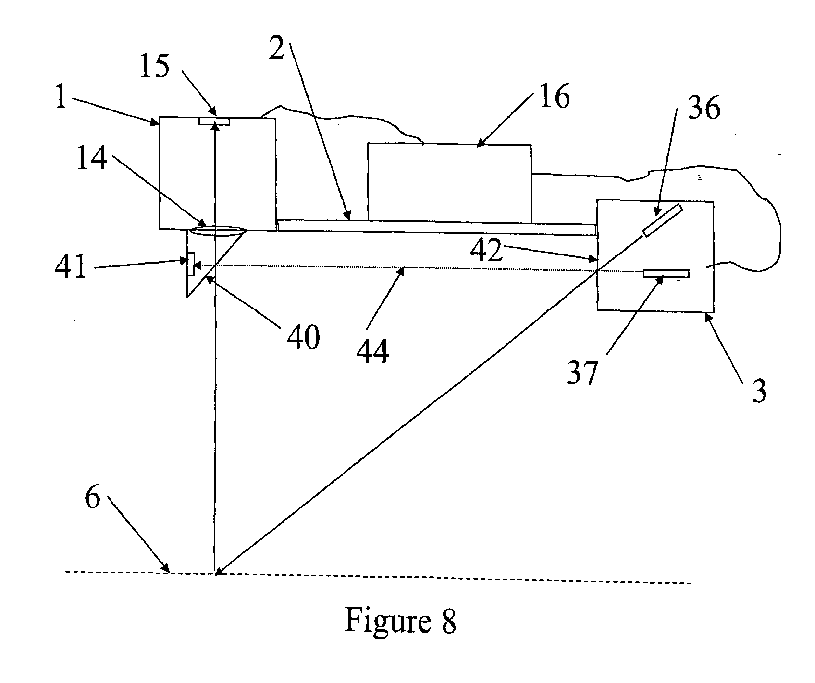

This arrangement cannot be used with the fixed radiation source units for example shown in figures 2 and 3 but a similar correction for window fouling may be derived using the arrangement of figure 8. Referring to figure 8, the fixed radiation source unit 3 includes one or more radiation sources 36 for the measurement of the sample characteristics as in for example figures 2 and 3 and the radiation detected from the interaction between radiation and sample is measured using detector unit 1. In so doing the radiation passes through window 42 in the radiation source unit 3 and the inclined window 40 in the detection unit 1. An auxiliary radiation source 37 is arranged in the radiation source unit 3 so that its collimated beam 44 passes through the windows 42 and 40 at the same point as the radiation from the source 36 when making measurements on the sample, and it is detected by auxiliary detector 41. This measurement will be attenuated by fouling of the windows 42 and 40 and can be used by the computer 16 to correct for the losses due to fouling on the windows.

In the embodiments of figures 7 and 8 the auxiliary detector 41 can be replaced by a small reflecting sphere or thin wire placed inside the window 40. Such a wire would scatter radiation into the main detector 15 when the collimated source beam 5 of figure 7 or the auxiliary collimated beam 44 of figure 8 strikes the wire. The measured intensity at the main detector 15 would thus provide data from which a correction for window fouling can be made. In the arrangement of figure 7 the wire or sphere would also provide registration for the swivelling collimated beam 5. Whilst the wire or sphere would obscure a portion of the window 40 the wire or sphere would be made sufficiently small so that the losses of scattered and fluorescent light from the sample 6 received at the main detector 15 were negligible.

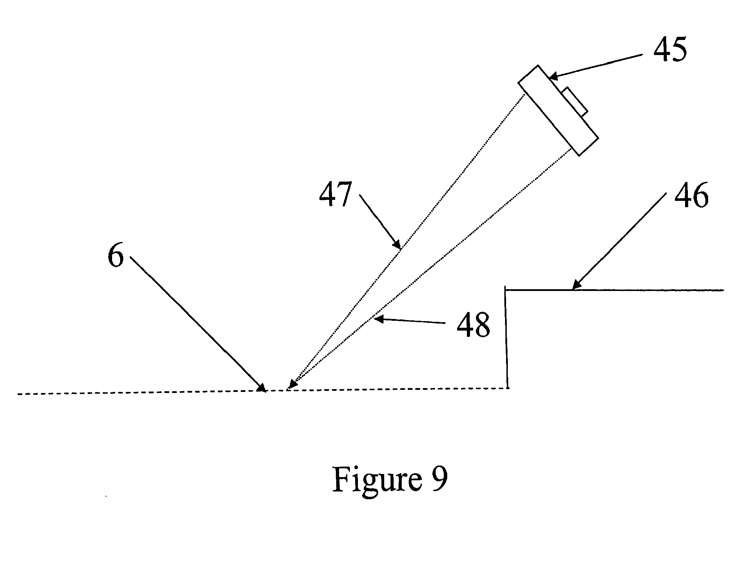

The invention may be used in handheld instrumentation. Referring to figure 9, the system is configured as a handheld unit 45 which is aimed at the sample surface 6 by the operator standing on the side of the channel 46. The optical axis 47 of the radiation source and the detector optical axis 48 are arranged to intersect at a range which is the middle of the expected range of operation.

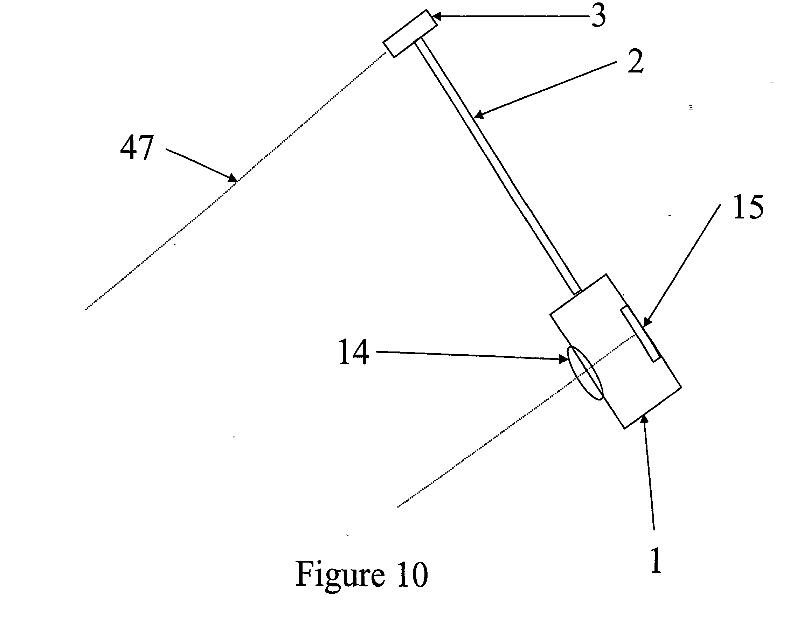

Referring to figure 10, the handheld unit has a radiation source unit 3 connected by a rigid arm 2 to a detector unit 1. It is convenient for the arm 2 to be telescopic or hinged to enable the system to be stored and carried in a compact form. The lens 14 focuses the radiation received on the detector 15 which is a position sensitive device or an array of detectors. By triangulation the range to the sample surface can be deduced from the position of the image of the radiation source on the sample surface, which moves laterally across the detector 15 as the range to the sample varies. In order to make correct measurements of the sample properties it is necessary to know the angle made by the source beam 47 and the sample surface. The system therefore includes an inclinometer which is connected with the radiation source and detector to a computer also included in the handheld system.

The lens 14 in all of the examples given may be for example a fixed focus lens. For large ranges of sample level it may be necessary to replace this fixed focus lens with a variable focus lens under computer control or a self-contained automatic focus system.

The present invention may be used for a range of possible measurements. An example is the measurement of the polluting load of a wastewater. In this application, two radiation sources are used, the first at a red or infrared wavelength in the range 670 to 880 nanometres the second in the far blue or near ultraviolet in the range 360 to 410 nanometres. Using these radiation sources, three measurements are made with the detection unit, the scattered radiation at the blue/near ultraviolet, the scattered radiation at the red/near infrared, and the fluorescence of the sample using the blue/near ultraviolet radiation as excitation and measuring the emission in the range 570 to 630 nanometres wavelength. It is found that for many wastewater samples a correlation exists between the suspended solids of the sample and the linear sum of the red/infrared scattered radiation (IR scatter) and the blue/near ultraviolet scattered radiation (UV scatter) of the form:

$$\text{Suspended Solids} = a_1 \times (\text{IR scatter}) + b_1 \times (\text{UV scatter}) + c_1$$

where $a_1$, $b_1$ and $c_1$ are constants to be determined for a given wastewater.

It has also been found that for many wastewaters a correlation exists between the BOD and COD of a sample and the linear sum of the red/infrared scattered radiation (IR scatter), the blue/near ultraviolet scattered radiation (UV scatter) and the fluorescence of the sample using the blue/near ultraviolet radiation as excitation and measuring the emission in the range 570 to 630 nanometres (UV-red fluorescence) of the form:

$$\text{BOD} = a_2 \times (\text{IR scatter}) + b_2 \times (\text{UV scatter}) + c_2 \, (\text{UV-red fluorescence}) + d_2$$

$$\text{COD} = a_3 \times (\text{IR scatter}) + b_3 \times (\text{UV scatter}) + c_3 \, (\text{UV-red fluorescence}) + d_3$$

where $a_2$, $b_2$, $c_2$, $a_3$, $b_3$ and $c_3$ are constants to be determined for a given wastewater.

For some wastewaters it may be advantageous to add non-linear terms to these expressions for suspended solids, BOD and COD.

It has further been found that for wastewater samples of similar origin the constants in the expressions for suspended solids, BOD and COD are very close to each other, thus enabling standard calibrations to be used. Samples may then be taken for analysis and used to trim the values of the standard constants.

The size of the suspended material and its quality will be influenced by the flow velocity. Where the present invention includes a flow velocity measurement or where it is available as an input to the system computer, this can be added to the above correlation expressions to obtain an improved prediction of the pollution parameters.

Some applications generate spurious high values for the scatter and fluorescence data caused by for example large ripples, foam and highly reflecting floating objects. The data can be smoothed by removing these spurious data values using for example a median filter.

It will be appreciated that the invention described above may be modified within the scope of the appended claims.

**Claims**

1. A device (1) for monitoring one or more characteristics of a liquid sample, comprising at least one source (3) of collimated radiation, and at least one detector (15) capable of detecting radiation returning from the sample, wherein the optical axis of the radiation source or sources and the optical axis of the detector or detectors are non-parallel, **characterized in that** the device further comprises means to correct for changes in the intensity of radiation received by the or each detector caused by variations in the range of the device from the sample surface, and variations in the incident angle and scatter angle of the collimated radiation, whereby the or each characteristic of the liquid can still be monitored even when there is a change in sample level.

2. A device according to claim 1, comprising at least two sources of collimated radiation arranged side by side or arranged on a common optical axis.

3. A device according to claim 1 or 2, comprising at least two radiation detectors arranged side by side or arranged on a common optical axis.

4. A device according to any preceding claim, wherein the or each collimated radiation source is a laser.

5. A device according to any preceding claim, further comprising means to enable the device to be held by hand, whereby the device is portable and can be used by handheld operation.

6. A device according to claim 5, further comprising means to measure the range from the device to the sample surface, and wherein the range measuring means is preferably a position sensitive detector.

7. A device according to claim 6, further comprising an inclinometer.

8. A device according to any preceding claim, wherein the radiation source is pivotable and controllable such that the interaction between radiation and sample occurs on or close to the optical axis of the radiation detector.

9. A device according to any one of claims 1 to 7, wherein the angle between the axis of the collimated radiation and the detector optical axis is controlled by the device using a mirror on a motorised rotating shaft such that the interaction between radiation and sample occurs on or close to the optical axis of the radiation detector

10. A device according to any preceding claim, wherein the detector consists of a linear array of detectors, or a single position sensitive detector such that as the level varies the image of the region of interaction of the source radiation and sample moves laterally across the detector.

11. A device according to any preceding claim, wherein the radiation source and radiation detector are mounted together on a float (22) which is free to move on one or more swinging arms or tethers (23) maintaining a constant range between the radiation source and detectors and the sample surface.

12. A device according to any preceding claim, further comprising means for measuring losses across the optical windows of the device.

13. A device according to claim 12, wherein the means for measuring the losses across the optical windows includes a detector window arranged such that collimated radiation from the radiation source can be passed through the radiation source window and detector optical window directly, without interaction with the sample..

14. A device according to claim 1, comprising a radiation source housing containing the or each radiation source (3), a detector housing containing the or each detector (15) and a lens (14) arranged such that radiation from the or each radiation source must pass through the lens in order to reach the or each detector, a support (2) for supporting the detector housing and the radiation source housing, and control means (16) for controlling the or each radiation source and the or each detector.

**15.** A method of determining the polluting properties of an aqueous sample using a device according to any preceding claim, the method comprising directing a beam of collimated radiation from a collimated radiation source of the device at the aqueous sample, detecting radiation returned back from the sample using a detector of the device, and processing the data received by the detector.

**16.** A method according to claim 15, wherein the aqueous sample comprises a sewage stream flowing along an open channel.

**17.** A method according to claim 15 or 16, wherein the suspended solids content of the aqueous sample is inferred from measurements of scattered radiation at one or more wavelengths.

**18.** A method according to claim 15, 16 or 17, wherein measurements of fluorescence using excitation in the range 360 - 410 nanometres and emission in the range 570 - 630 nanometres are used to infer the organic load of the aqueous sample which may be expressed as biochemical oxygen demand or chemical oxygen demand.

**Patentansprüche**

**1.** Eine Vorrichtung (1) zur Überwachung einer oder mehrerer Eigenschaften einer flüssigen Probe, umfassend mindestens eine Quelle (3) von kollimierter Strahlung und mindestens einen Detektor (15), geeignet zur Detektion von Strahlung, welche von der Probe zurückkehrt, worin die optische Achse der Strahlenquelle oder der Strahlenquellen und die optische Achse des Detektors oder der Detektoren nicht parallel sind, **dadurch gekennzeichnet, dass** die Einrichtung weiter Mittel zur Korrektur von Änderungen in der Intensität der von dem oder jedem Detektor empfangenen Strahlung umfasst, welche durch Variationen in der Entfernung der Einrichtung von der Probenoberfläche und durch Variationen in dem Einfalls-und Streuwinkel der kollimierten Strahlung verursacht werden, wodurch die oder jede Eigenschaft der Flüssigkeit sogar bei einer Änderung des Probenniveaus weiterhin überwacht werden kann.

**2.** Eine Vorrichtung nach Anspruch 1, umfassend mindestens zwei Quellen kollimierte Strahlung, welche Seite an Seite oder auf einer gewöhnlichen optischen Achse angeordnet sind.

**3.** Eine Vorrichtung nach Anspruch 1 oder 2, umfassend mindestens zwei Strahlungsdetektoren, welche Seite an Seite oder auf einer gewöhnlichen optischen Achse angeordnet sind.

**4.** Eine Vorrichtung nach einem der vorherigen Ansprüche, worin die oder jede Quelle kollimierter Strahlung ein Laser ist.

**5.** Eine Vorrichtung nach einem der vorherigen Ansprüche, weiter Mittel umfassend, welche es ermöglichen, die Vorrichtung mit der Hand zu halten, wodurch die Vorrichtung tragbar ist und zum handgehaltenen Betrieb verwendet werden kann.

**6.** Eine Vorrichtung nach Anspruch 5, weiter umfassend Mittel zur Messung der Entfernung von der Vorrichtung zu der Probenoberfläche und worin die Mittel zur Entfernungsmessung vorzugsweise ein positionsempfindlicher Detektor sind.

**7.** Eine Vorrichtung nach Anspruch 6, weiter ein Inklinometer umfassend.

**8.** Eine Vorrichtung nach einem der vorherigen Ansprüche, worin die Strahlungsquelle drehbar und steuerbar ist, sodass die Wechselwirkung zwischen Strahlung und Probe in oder nahe der optischen Achse des Strahlungsdetektors auftritt.

**9.** Eine Vorrichtung nach einem der Ansprüche 1 bis 7, worin der Winkel zwischen der Achse der kollimierten Strahlung und der optischen Achse des Detektors von der Einrichtung unter Verwendung eines Spiegels an einer motorisiert drehenden Welle gesteuert wird, sodass die Wechselwirkung zwischen Strahlung und Probe in oder nahe der optischen Achse des Strahlungsdetektors auftritt.

**10.** Eine Vorrichtung nach einem der vorherigen Ansprüche, worin der Detektor aus einer linearen Anordnung von Detektoren oder einem einzelnen positionsempfindlichen Detektor besteht, sodass sich beim Ändern des Niveaus das Bild des Bereichs der Wechselwirkung der Quellenstrahlung und der Probe seitwärts über den Detektor bewegt.

11. Eine Vorrichtung nach einem der vorherigen Ansprüche, worin die Strahlungsquelle und der Strahlungsdetektor zusammen auf einem Schwimmkörper (22) befestigt werden, welcher an einem oder mehreren Schwingarmen oder Halteseilen (23) frei beweglich ist zur Aufrechterhaltung einer konstanten Entfernung zwischen der Strahlungsquelle, der Strahlungsdetektoren und der Probenoberfläche.

12. Eine Vorrichtung nach einem der vorherigen Ansprüche, weiter umfassend Mittel zur Messung von Verlusten quer durch die optischen Fenster der Vorrichtung.

13. Eine Vorrichtung nach Anspruch 12, worin die Mittel zur Messung der Verluste quer durch die optischen Fenster ein Detektorfenster einschließen, welches so angeordnet ist, dass kollimierte Strahlung direkt und ohne eine Wechselwirkung mit der Probe von der Strahlungsquelle durch das Fenster der Strahlungsquelle und das optische Fenster des Detektors geführt werden kann.

14. Eine Vorrichtung nach Anspruch 1, umfassend ein Strahlungsquellengehäuse enthaltend die oder jede Strahlungsquelle (3), ein Detektorgehäuse enthaltend den oder jeden Detektor (15), und eine Linse (14), so angeordnet, dass Strahlung von der oder jeder Strahlungsquelle durch die Linse treten muss, um den oder jeden Detektor zu erreichen, eine Halterung (2) zum Halten des Detektorgehäuses und des Strahlungsquellengehäuses, und Steuerungsmittel (16) zur Steuerung der oder jeder Strahlungsquelle und dem oder jedem Detektor.

15. Ein Verfahren zur Bestimmung der Verschmutzungseigenschaften einer wässrigen Probe mit Nutzung einer Vorrichtung nach einem der vorhergehenden Ansprüche, wobei das Verfahren umfasst: Lenken eines Strahls von kollimierter Strahlung von einer Quelle kollimierter Strahlung der Vorrichtung auf die wässrige Probe, Detektieren der von der Probe zurückkehrenden Strahlung unter Verwendung eines Detektors der Vorrichtung und Verarbeiten der vom Detektor empfangenen Daten.

16. Ein Verfahren nach Anspruch 15, worin die wässrige Probe einen Abwasserstrom umfasst, welcher in einem offenen Kanal fließt.

17. Ein Verfahren nach Anspruch 15 oder 16, worin der Gehalt an suspendierten Feststoffen in der wässrigen Probe aus Messungen von gestreuter Strahlung bei einer oder mehrerer Wellenlängen gefolgert wird.

18. Ein Verfahren nach Anspruch 15, 16 oder 17, worin Messungen der Fluoreszenz unter Verwendung von Anregung im Bereich von 360 - 410 Nanometern und von Emission im Bereich von 570 - 630 Nanometern zum Folgern der organischen Belastung benutzt werden, welche als biochemischer Sauerstoffbedarf oder als chemischer Sauerstoffbedarf ausgedrückt werden können.

**Revendications**

1. Dispositif (1) pour contrôler une ou plusieurs caractéristiques d'un échantillon liquide comprenant au moins une source (3) de rayonnement collimaté et au moins un détecteur (15) capable de détecter le rayonnement retournant de l'échantillon, dans lequel l'axe optique de la source ou des sources de rayonnement et l'axe optique du détecteur ou des détecteurs sont non parallèles, **caractérisé en ce que** le dispositif comprend, en outre, un moyen pour corriger les modifications dans l'intensité de rayonnement reçu par le ou chaque détecteur provoqué par les variations dans la gamme du dispositif à partir de la surface de l'échantillon et les variations dans l'angle incident et l'angle de dispersion du rayonnement collimaté de sorte que la ou chaque caractéristique du liquide peut encore être contrôlée, même lorsqu'il y a une modification dans le niveau d'échantillon.

2. Dispositif selon la revendication 1, comprenant au moins deux sources de rayonnement collimaté agencées côte à côte ou agencées sur un axe optique commun.

3. Dispositif selon l'une des revendications 1 ou 2, comprenant au moins deux détecteurs de rayonnement agencés côte à côte ou agencés sur un axe optique commun.

4. Dispositif selon l'une quelconque des revendications précédentes, dans lequel le ou chaque source de rayonnement collimaté est un laser.

**5.** Dispositif selon l'une quelconque des revendications précédentes, comprenant, en outre, un moyen permettant au dispositif d'être maintenu à la main de sorte que le dispositif est portable et peut être utilisé par une opération de retenue à la main.

**6.** Dispositif selon la revendication 5, comprenant, en outre, un moyen pour mesurer la gamme du dispositif à la surface de l'échantillon et dans lequel le moyen de mesure de la gamme est, de préférence, un détecteur sensible de position.

**7.** Dispositif selon la revendication 6, comprenant, en outre, un inclinomètre.

**8.** Dispositif selon l'une quelconque des revendications précédentes, dans lequel la source de rayonnement est apte à pivoter et apte à être commandée de sorte que l'interaction entre le rayonnement et l'échantillon se produit sur l'axe optique ou près de l'axe optique du détecteur de rayonnement.

**9.** Dispositif selon l'une quelconque des revendications 1 à 7, dans lequel l'angle entre l'axe du rayonnement collimaté et l'axe optique du détecteur est commandé par le dispositif utilisant un miroir sur un arbre tournant motorisé de sorte que l'interaction entre le rayonnement et l'échantillon se produit sur l'axe optique ou près de l'axe optique du détecteur de rayonnement.

**10.** Dispositif selon l'une quelconque des revendications précédentes, dans lequel le détecteur comprend un réseau linéaire de détecteurs ou un seul détecteur sensible de position de sorte que comme le niveau modifie l'image de la région d'interaction du rayonnement de la source, l'échantillon se déplace latéralement au niveau du détecteur.

**11.** Dispositif selon l'une quelconque des revendications précédentes, dans lequel la source de rayonnement et le détecteur de rayonnement sont montés conjointement sur un flotteur (22) qui est libre de se déplacer sur un ou plusieurs bras de pivotement ou attaches (23) maintenant une gamme constante entre la source de rayonnement et les détecteurs et la surface de l'échantillon.

**12.** Dispositif selon l'une quelconque des revendications précédentes, comprenant, en outre, un moyen pour mesurer les pertes au niveau des fenêtres optiques du dispositif.

**13.** Dispositif selon la revendication 12, dans lequel le moyen pour mesurer les pertes au niveau des fenêtres optiques comprend une fenêtre de détecteur agencée afin que le rayonnement collimaté à partir de la source de rayonnement puisse traverser la fenêtre de la source de rayonnement et la fenêtre optique du détecteur directement, sans interaction avec l'échantillon.

**14.** Dispositif selon la revendication 1, comprenant un logement de source de rayonnement contenant la ou chaque source de rayonnement (3), un logement de détecteur contenant le ou chaque détecteur (15) et une lentille (14) agencée afin que le rayonnement à partir de la ou chaque source de rayonnement puisse traverser la lentille pour atteindre le ou chaque détecteur, un support (2) pour supporter le logement du détecteur et le logement de la source de rayonnement et un moyen de commande (16) pour commander la ou chaque source de rayonnement et le ou chaque détecteur.

**15.** Procédé pour déterminer les propriétés de pollution d'un échantillon aqueux utilisant un dispositif selon l'une quelconque des revendications précédentes, le procédé comprenant la direction d'un faisceau de rayonnement collimaté à partir d'une source de rayonnement collimaté du dispositif au niveau de l'échantillon aqueux, la détection du rayonnement ramené à partir de l'échantillon en utilisant un détecteur du dispositif et le traitement des données reçues par le détecteur.

**16.** Procédé selon la revendication 15, dans lequel l'échantillon aqueux comprend un courant d'eaux usées s'écoulant le long d'un canal ouvert.

**17.** Procédé selon la revendication 15 ou 16, dans lequel la teneur des solides en suspension de l'échantillon aqueux est déduite des mesures du rayonnement diffusé à une ou plusieurs longueurs d'onde.

**18.** Procédé selon la revendication 15, 16 ou 17, dans lequel des mesures de la fluorescence utilisant l'excitation dans la gamme de 360-410 nanomètres et l'émission dans la gamme de 570-630 nanomètres sont utilisées pour déduire la charge organique de l'échantillon aqueux qui peut être exprimée comme une demande d'oxygène biochimique ou une demande d'oxygène chimique.

Figure 1

Figure 2

Plan

22

15

14

1    27

3

24

28

Figure 3

23

26

25

Elevation

EP 1 241 464 B1

Figure 4

EP 1 241 464 B1

Plan

Elevation

Figure 5

EP 1 241 464 B1

Figure 6

Figure 7

Figure 8

EP 1 241 464 B1

Figure 9

EP 1 241 464 B1

Figure 10

EP 1 241 464 B1

**EP 1 241 464 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3309956 A
- US 5400137 A
- GB 2310282 A
- US 4668860 A